# EUROPEAN PATENT SPECIFICATION

(11) **EP 1 935 363 B1**
(45) Date of publication and mention of the grant of the patent: **24.06.2015**
(21) Application number: 07023560.1
(22) Date of filing: 05.12.2007
(51) Int. Cl.: A61B 19/00

(54) **Organ anastomosis marker, marker positioning apparatus and transendoscopic gastrojejunal anastomosis procedure**
Organ-Anastomose-Marker, Markerpositionierungsvorrichtung und transendoskopisches Gastrojejunal-Anastomose-Verfahren
Marqueur d'anastomose d'organes, appareil de positionnement de marqueur et procédé d'anastomose gastro-jéjunale transendoscopique

(30) Priority: 21.12.2006 JP 2006345060
(43) Date of publication of application: 25.06.2008
(73) Proprietor: OLYMPUS MEDICAL SYSTEMS CORP., Tokyo 151-0072 (JP)
(72) Inventor: Mizunuma, Akiko, Tokyo 151-0072 (JP)
(74) Representative: von Hellfeld, Axel

(56) References cited:
- WO-A-03/077730
- WO-A-2004/045480
- US-A- 5 759 154
- US-A- 6 106 473
- US-B1- 6 371 904

## Description

### BACKGROUND OF THE INVENTION

### 1. Field of the Invention

The present invention relates to an organ anastomosis marker provided with marker portions displayed on an ultrasound tomographic image, a marker positioning apparatus for positioning the marker portions in a body cavity, and a transendoscopic gastrojejunal anastomosis procedure using the organ anastomosis marker.

### 2. Description of the Related Art

Gastrojejunal anastomosis is performed as a laparotic procedure for symptomatic treatment of duodenal obstruction. In addition, gastrojejunal anastomosis is also performed as a laparoscopic procedure. However, these procedures are highly invasive for patients and are difficult to be applied to patients such as terminal cancer patients having depleted physical strength. On the other hand, gastrojejunal anastomosis is attracting attention in the U.S. and Europe as a treatment method for extreme obesity.

The use of a transendoscopic procedure has recently been proposed for application to gastrojejunal anastomosis for the purpose of reducing the physical burden on patients.

Japanese Patent Application Laid-open No. 2004-267772 discloses an endoscopic intraabdominal treatment system. In this system, an orally inserted endoscope can be transgastrically inserted into the abdominal cavity to perform treatment within the abdominal cavity by emitting light from a distal end portion of an illuminator.

In the case of performing gastrojejunal anastomosis transendoscopically, it is difficult to determine the optimum puncture site and puncture direction based on endoscopic images alone. Consequently, an ultrasound endoscope has been proposed for use as an endoscope for performing gastrojejunal anastomosis transendoscopically with which deep blood vessels or organs are displayed on an ultrasound tomographic image, and puncturing is performed under the guidance of the ultrasound endoscope.

However, in addition to the jejunum being positioned in the form of a complex structure within the abdominal cavity, there are also considerable individual differences in terms of the anatomy thereof. Namely, it is difficult to clearly distinguish the target site in the form of the jejunum, the large intestine or ileum on ultrasound tomographic images.

With the foregoing in view, an object of the present invention is to provide an organ anastomosis marker that makes it possible to reliably perform puncture by specifying a target site from the stomach to the jejunum under the guidance of an ultrasound endoscope.

WO 03/077730 A2 discloses an ultrasonic marker according to the preamble of claim 1.

### SUMMARY OF THE INVENTION

The invention relates to the organ anastomosis marker as defined by claim 1.

The above and other objects, features and advantages of the invention will become more clearly understood from the following description referring to the accompanying drawings.

### BRIEF DESCRIPTION OF THE DRAWINGS

FIG 1 is a drawing for explaining the relationship between an endoscope and a marker positioning apparatus introduced into an abdominal cavity via a treatment instrument channel of the endoscope;
FIG. 2 is an exploded perspective view for explaining an organ anastomosis marker, an apparatus body and an extruding member;
FIG 3 is a drawing for explaining a metal organ anastomosis marker provided with a plurality of circular grooves in the surface thereof;
FIG 4 is a cross-sectional view for explaining the configuration of a marker positioning apparatus composed of an organ anastomosis marker, an apparatus body and an extruding member;
FIG 5 is a drawing for explaining a target site where gastrojejunal anastomosis is to be performed and a state in which an endoscope is inserted into the duodenum;
FIG 6 is a perspective view for explaining the configuration of a distal end portion of an ultrasound endoscope;
FIG 7 is a front view of the ultrasound endoscope shown in FIG 6;
FIG 8 is a flow chart showing the procedure for transendoscopic gastrojejunal anastomosis;
FIGS. 9A and 9B are drawings for explaining the procedure for inserting a marker positioning apparatus into a body cavity via an endoscope insertion portion;

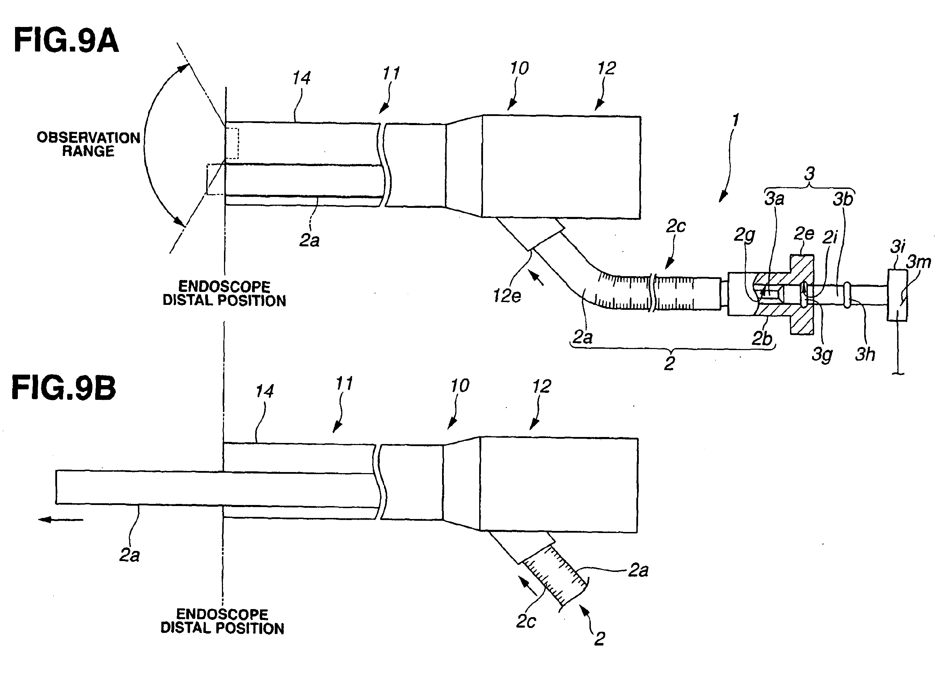

FIG. 9A is a drawing for explaining a state in which an insertion portion of a marker positioning apparatus is introduced from a treatment instrument inlet into a treatment instrument channel;
FIG 9B is a drawing for explaining a state in which a distal end surface of an insertion portion is inserted by reading a scale;

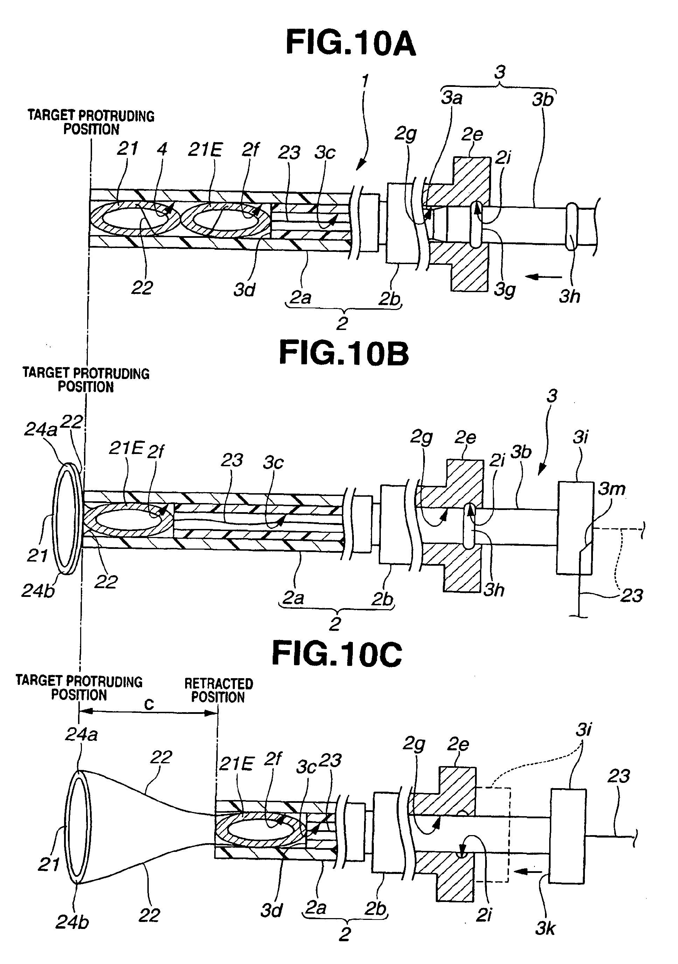

FIGS. 10A, 10B and 10C are drawings for explaining the procedure for positioning a marker portion housed in a marker housing space portion of a marker positioning apparatus in the jejunum;
FIG 10A is a drawing showing a state in which a distal end surface of an insertion portion is positioned at a target protruding position;
FIG 10B is a drawing showing a state in which a first stage of an extruding operation on an extruding member is terminated;
FIG 10C is a drawing for explaining a state in which a distal end surface of an insertion member is retracted by a predetermined distance from a target protruding position, and a state in which the second stage of an extruding operation of an extruding member is completed;

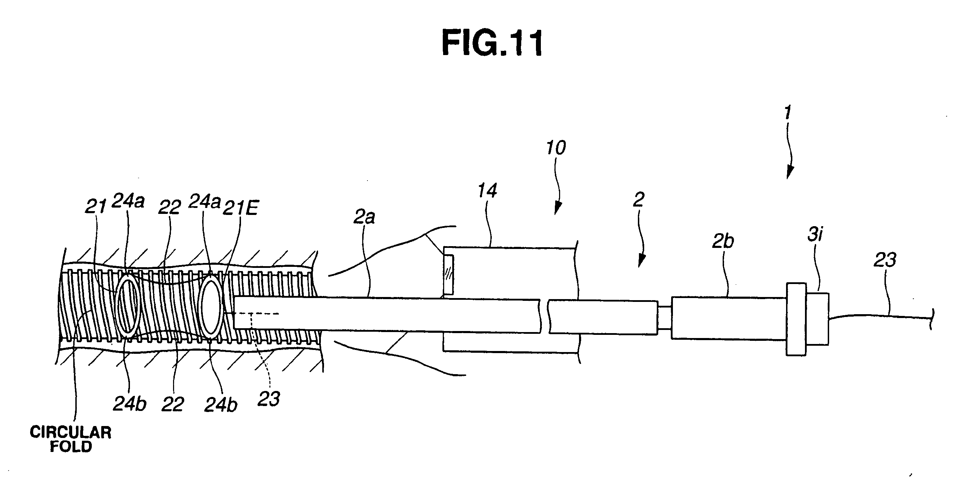

FIG 11 is a drawing for explaining a state in which two marker portions are positioned within the jejunum by a marker positioning apparatus;

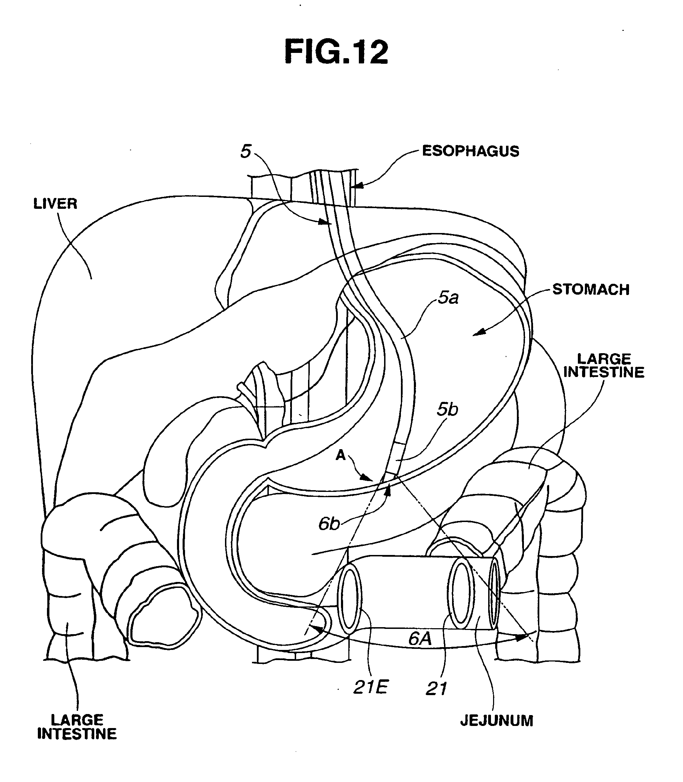

FIG 12 is a drawing for explaining a state in which two marker portions are captured within an ultrasound region of an ultrasound endoscope;

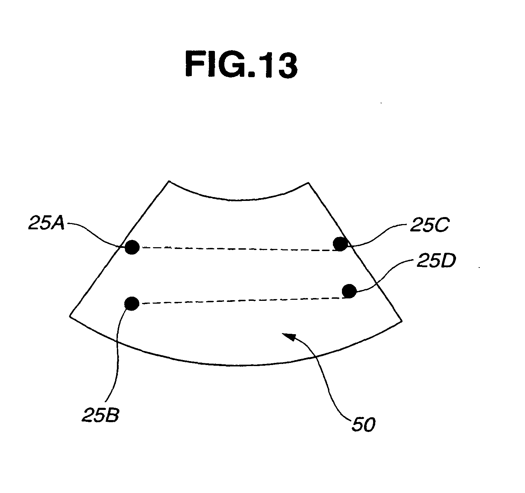

FIG 13 is a drawing showing an ultrasound tomographic image of two marker portions captured within an ultrasound region;

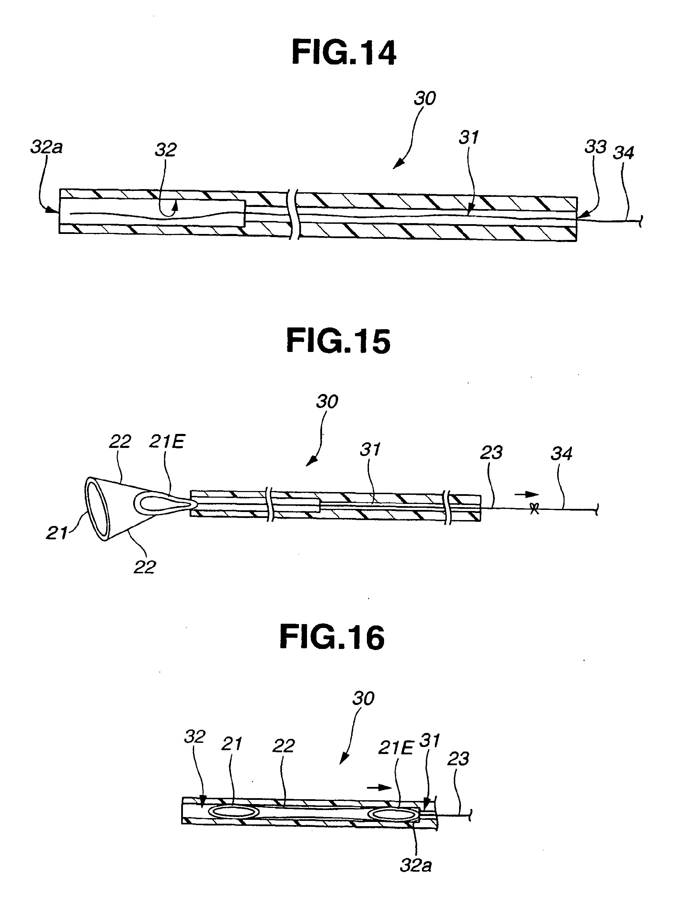

FIGS. 14 to 16 relate to a marker withdrawal instrument;
FIG 14 is a drawing for explaining the configuration of a marker withdrawal instrument;
FIG 15 is a drawing for explaining a state in which a marker portion is withdrawn by a marker withdrawal instrument:
FIG. 16 is a drawing showing a state in which a marker portion is housed in a housing portion of a marker withdrawal instrument;

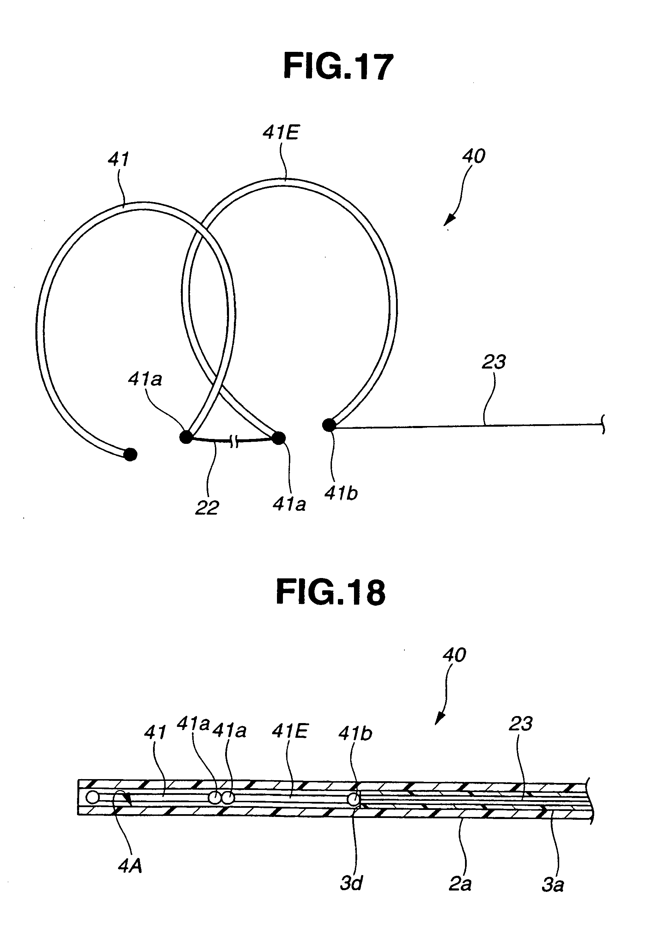

FIGS. 17 and 18 show a second embodiment of an organ anastomosis marker;
FIG 17 is a drawing for explaining another configuration of an organ anastomosis marker; and
FIG 18 is a cross-sectional view for explaining a state in which the organ anastomosis marker of FIG 17 is housed in a marker housing space portion.

### DETAILED DESCRIPTION OF PREFERRED EMBODIMENTS

The following provides an explanation of embodiments of the present invention with reference to the drawings.

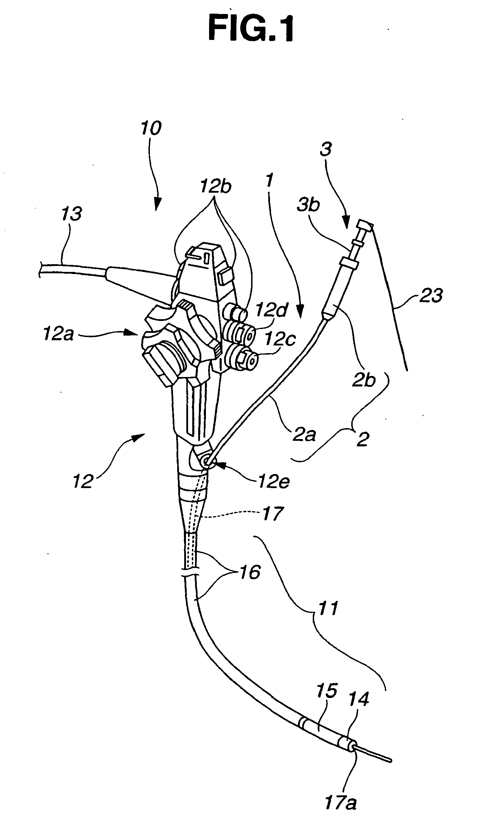

An explanation is first provided of a first embodiment of an organ anastomosis marker and marker positioning apparatus with reference to FIGS. 1 and 13.

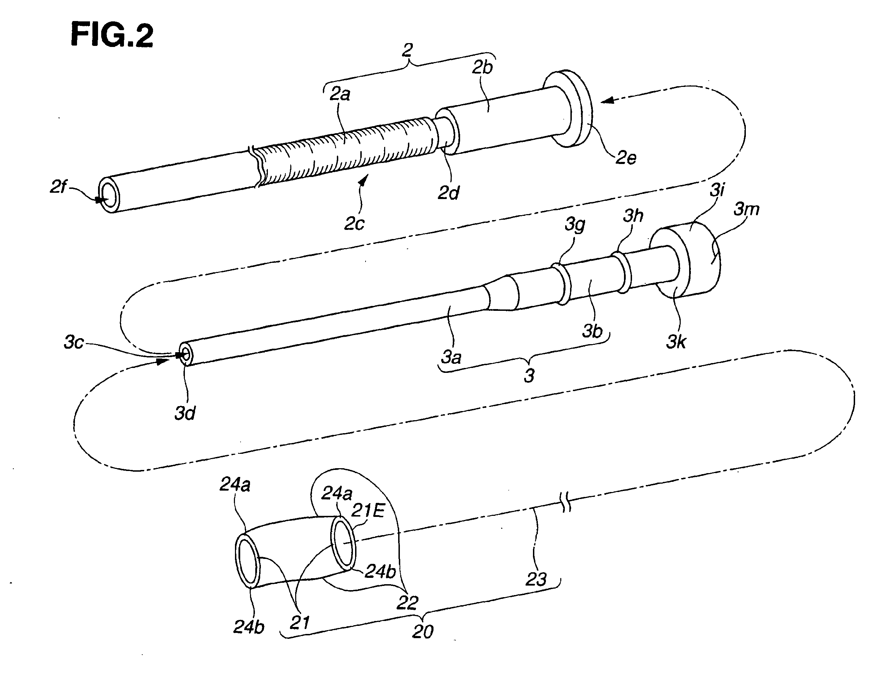

As shown in FIG. 1, a marker positioning apparatus (to be simply referred to as a positioning apparatus) 1 is composed of an apparatus body 2 and an extruding member 3. The apparatus body 2 is composed of an insertion portion 2a and a barrel 2b. The insertion portion 2a is composed of a tube body, and a plurality of marker portions 21 of the organ anastomosis marker (see reference symbol 20 in FIG 2) to be described later are housed in the distal end side thereof. The barrel 2b is connected to the proximal end portion of the insertion portion 2a.

An extruding member 3 is composed of a marker guiding portion (reference symbol 3a in FIG 2) and a tube 3b. The marker guiding portion 3a is composed of a tube body, and the marker guiding portion 3a is slidably positioned within a through hole of the insertion portion 2a constituting the apparatus body 2. The tube 3b is connected to the proximal end portion of the marker guiding portion 3a.

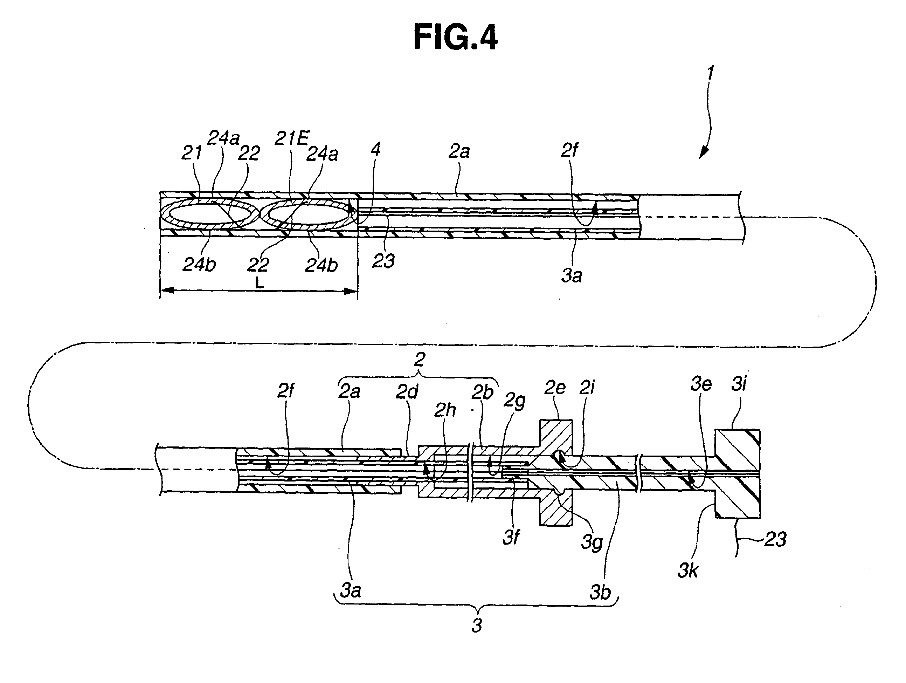

The insertion portion 2a and the marker guiding portion 3 a are flexible. The insertion portion of the marker positioning apparatus 1 (see reference symbol 1A of FIG. 4) when the marker guiding portion 3a has been inserted in the insertion portion 2a is introduced into a body cavity via a treatment instrument channel 17 provided in an endoscope 10 to be described later. Furthermore, reference symbol 23 indicates a string-like member to be described later.

The endoscope 10 is composed of an endoscope insertion portion 11 inserted into a body cavity, an operation portion 12, and a universal cord 13. The endoscope insertion portion 11 is long and has a narrow diameter. The operation portion 12 is connected to the proximal end side of the endoscope insertion portion 11. The universal cord 13 extends from one side of the operation portion 12.

The endoscope insertion portion 11 is composed of a distal end rigid portion 14, a bending portion 15 and a flexible tube portion 16 in that order starting from the distal end side thereof. An image pickup unit (not shown), illuminating optics and the like are contained within the distal end rigid portion 14. The image pickup unit is composed of an image pickup means in the form of an image pickup device such as a CCD or CMOS, observation optics and the like. The illuminating optics are positioned facing a distal end surface of a light guide or a front surface of an optical device that supplies illumination light for illuminating a target observation site in a body cavity. An observation window not shown that constitutes the observation optics, an illumination window not shown that constitutes the illumination optics and a distal end opening 17a of the treatment instrument channel 17 and the like are provided on the distal end surface of the distal end rigid portion 14.

The operation portion 12 is provided with a bending operation knob 12a, various switches 12b for instructing the image pickup means to freeze or release and the like, an air/water feed button 12c, and an aspiration button 12d and the like. Reference symbol 12e indicates a treatment instrument inlet.

An endoscope connector is provided on an end portion not shown of the universal cord 13. The endoscope connector is removably connected to external apparatuses in the form of a light source apparatus and a video processor. An electrical connector for electrically connecting to an external apparatus in the form of an ultrasound diagnostic apparatus is provided in the endoscope connector.

The following provides an explanation of the organ anastomosis marker (to be simply referred to as the marker) 20 and the positioning apparatus 1.

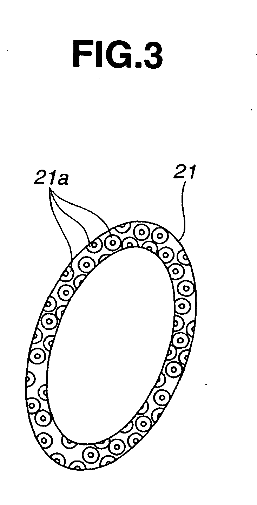

An explanation of the marker 20 is provided with reference to FIGS. 2 and 3.

As shown in FIG 2, the marker 20 is composed of a plurality, for example two, of the marker portions 21, a pair of string-like connecting threads 22, and an operation thread 23. The marker portions 21 are made of an elastic member that reflects ultrasound waves in the form of a metal such as tungsten or an ultra-flexible resin such as Grilamid, and are circular in the no-load state as shown in the drawing. In the case the marker portions 21 are made of metal, a plurality of circular grooves 21 a are provided at a high density in the surface of the marker portions 21 to reflect ultrasound waves as shown in FIG. 3. On the other hand, in the case the marker portions 21 are made of resin, an ultrasound wave-reflecting substance such as ferrite, tungsten powder, alumina powder, glass beads, glass fibers or carbon fibers are mixed into the resin material that forms the marker portions 21. The connecting threads 22 are connecting members that connect the two marker portions 21. The operation thread 23 is a string-like member such as surgical thread.

The connecting threads 22 are connected to each of the marker portions 21 so that the interval between the two marker portions 21 is a preset interval. In the present embodiment, the positions at which each end of the connecting threads 22 is fixed are set to be in a positional relationship that is symmetrical about the center of each of the marker portions 21. Namely, a line that connects a fixed portion 24a and a fixed portion 24b is substantially equal to the diameter of the ring.

The operation thread 23 is fixed only to one of the marker portions 21. The specific fixing position of the operation thread 23 is substantially the intermediate position between the fixed portions 24a and 24b of the two connecting threads 22. Furthermore, in the present embodiment, the marker portion 21 to which the operation thread 23 is fixed is indicated with reference symbol 21E.

The following provides an explanation of the apparatus body 2 and the extruding member 3 with reference to FIGS. 2 and 4.

As shown in FIG 2, the apparatus body 2 is composed of the insertion portion 2a and the barrel 2b. The outer diameter dimension of the insertion portion 2a is set to a dimension that allows insertion into the treatment instrument channel 17 of the endoscope 10. A scale 2c for visually confirming an introduced amount into a body cavity is provided on the outer surface of the handheld side of the insertion portion 2a.

On the other hand, a connecting portion 2d is provided on the distal end side of the barrel 2b where the proximal end portion of the insertion portion 2a is fixed. In addition, a barrel flange portion 2e is provided on the proximal end side of the barrel 2b for hooking the fingers. Reference symbol 2f shown in FIGS. 2 and 4 indicates an insertion portion through hole of the insertion portion 2a. As shown in FIG 4, a tube positioning hole 2g is provided in the barrel 2b in which the tube 3b is slidably positioned. Reference symbol 2h indicates a communicating hole that connects the tube positioning hole 2g with the outside on the side of the distal end surface of the connecting portion 2d, and the marker guiding portion 3a is inserted into the communicating hole 2h.

Furthermore, although the inner diameter dimension of the communicating hole 2h is narrower than the inner diameter dimension of the tube positioning hole 2g, the inner diameter dimension of the communicating hole 2h may be formed to the same diameter as the inner diameter dimension of the tube positioning hole 2g.

As shown in FIG 2, the extruding member 3 is composed of the marker guiding portion 3a and the tube 3b. The marker guiding portion 3a is slidably positioned within the insertion hole through hole 2f of the insertion portion 2a as shown in FIG 4.

As shown in FIGS. 2 and 4, the marker guiding portion 3a is provided with a guiding portion through hole 3c through which the operation thread 23 is inserted. The distal end surface of the marker guiding portion 3a is configured in the form of an extruding surface 3d that pushes out the marker portions 21.

On the other hand, the tube 3b is provided with a tube through hole 3e through which the operation thread 23 is inserted as shown in FIG 4. A connecting portion 3f is provided on the distal end side of the tube 3b where the proximal end portion of the marker guiding portion 3a is fixed.

As shown in FIG 2, two circular projections 3g and 3h are provided at predetermined locations on the outer surface of the tube 3b that are constituted by, for example, positioning two O-rings. In addition, a tube flange 3i is provided on the proximal end side of the tube 3b. The distal end surface of the tube flange 3i is configured in the form of a contact surface 3k, and contacts the proximal end surface of the barrel flange portion 2e. A slit 3m is formed in the tube flange portion 3i. The slit 3m is for fixing and retaining the operation thread 23 extending from the tube through hole 3e. The operation thread 23 is removable with respect to the slit 3m.

The distance from the first projection 3g to the contact surface 3k is set in consideration of a marker housing space portion 4 to be described later. On the other hand, the distance from the first projection 3g to the second projection 3h, and the distance from the second projection 3h to the contact surface 3k are set in consideration of the dimensions necessary to push out the marker portions 21 shown in FIG 4, housed while elastically deformed in the marker housing space portion 4, one at a time. The distance from the first projection 3g to the second projection 3h and the distance from the second projection 3h to the contact surface 3k are of equal dimensions.

Furthermore, reference symbol 2i in FIG. 4 indicates a circular indentation. The circular indentation 2i is provided at a predetermined location of the tube positioning hole 2g, and the projections 3f and 3g are engaged and positioned.

The following provides an explanation of an assembly procedure for the positioning apparatus 1.

The positioning apparatus 1 is constituted by assembling the apparatus body 2, the extruding member 3 and the marker 20 in, for example, the order indicated below.

First, a worker inserts the marker guiding portion 3a constituting the extruding member 3 into the insertion portion through hole 2f via the tube positioning hole 2g and the communicating hole 2h through a proximal end opening formed in the barrel flange portion 2e provided on the barrel 2b constituting the apparatus body 2. Subsequently, the tube 3b is inserted into the tube positioning hole 2g through the proximal end opening. The worker then positions first projection 3g provided on the outer surface of the tube 3b by engaging with the circular indentation 2i provided in the inner surface of the tube positioning hole 2g.

Once the projection 3g has been positioned in the indentation 2i, the extruding member 3 is held in the apparatus body 2 in a state in which the distal end surface of the marker guiding portion 3a is positioned towards the proximal end side by a predetermined dimension L from the distal end surface of the insertion portion 2a. Accordingly, the marker housing space portion 4 is formed on the distal end side of the insertion portion through the hole 2f of the insertion portion 2a.

Next, with the first projection 3g positioned in the circular indentation 2i, the worker inserts the operation thread 23 constituting the marker 20 into the insertion portion through hole 2f through the distal end opening of the insertion portion 2a constituting the apparatus body 2, and then inserts the operation thread 23 into the guiding portion through hole 3c through the distal end opening of the marker guiding portion 3a positioned in the insertion hole through hole 2f. Whereupon, after passing through the guiding portion through hole 3c of the marker guiding portion 3a, the operation thread 23 is inserted through the tube through hole 3e provided in the tube 3b and then subsequently led to the outside through the proximal end opening on the flange portion side of the tube 3b.

Here, the worker draws the operation thread 23 close to the handheld side. In addition, the worker positions the marker 21 E of the two marker portions 21 to which the operation thread 23 is fixed in the marker housing space portion 4 by deforming the marker portion 21 E in opposition to the resiliency thereof. Subsequently, the operation thread 23 is drawn even closer to the handheld side and the marker portion 21E is contacted with the extruding surface 3d of the marker guiding portion 3a. The worker then fixes and retains the operation thread 23 led out from the proximal end opening in the slit 3m and maintains the operation thread 23 in the state of being hooked on the slit 3m to retain the state of contact between the marker portion 21E and the extruding surface 3d of the marker guiding portion 3a.

Subsequently, the worker houses the connecting threads 22 in the marker housing space portion 4 together with positioning the other marker portion 21 in the marker housing space portion 4 by deforming in opposition to the resiliency thereof. As a result, the positioning apparatus 1 is configured as shown in FIG. 4. In the positioning apparatus 1, the tube 3b of the extruding member 3 is positioned in the tube positioning hole 2g provided in the barrel 2b of the apparatus body 2, and the two markers 21 and 2 1 E are housed in the marker housing space portion 4.

The positioning apparatus 1 configured in this manner is then supplied to a user while housed in a sterile pouch.

The following provides an explanation of the operation of the position apparatus 1 configured in the manner described above.

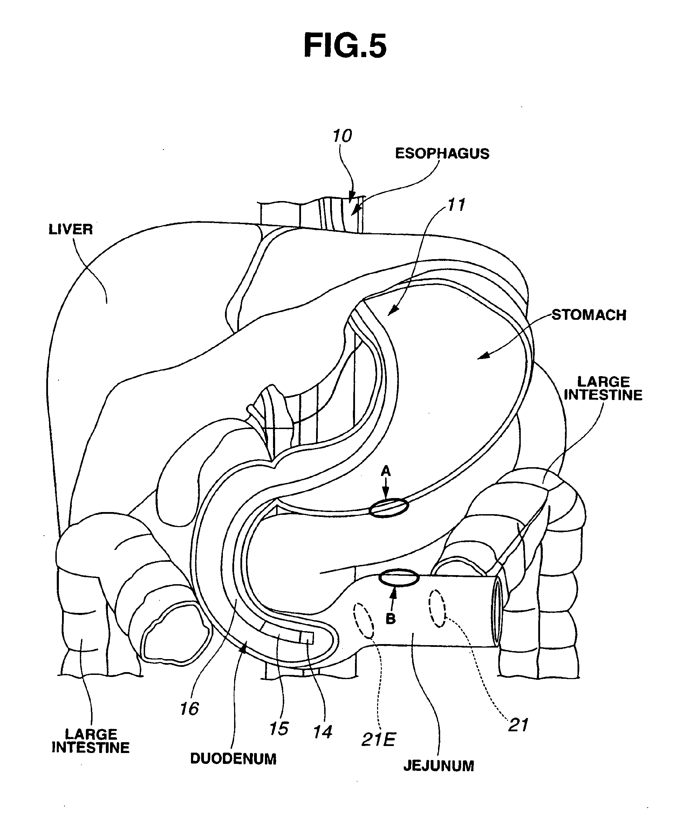

In performing transendoscopic gastrojejunal anastomosis in which a target site A of the stomach and a target site B of the jejunum shown in FIG 5 are anastomosed, the user prepares the positioning apparatus 1, the endoscope 10 and an ultrasound endoscope to be described below.

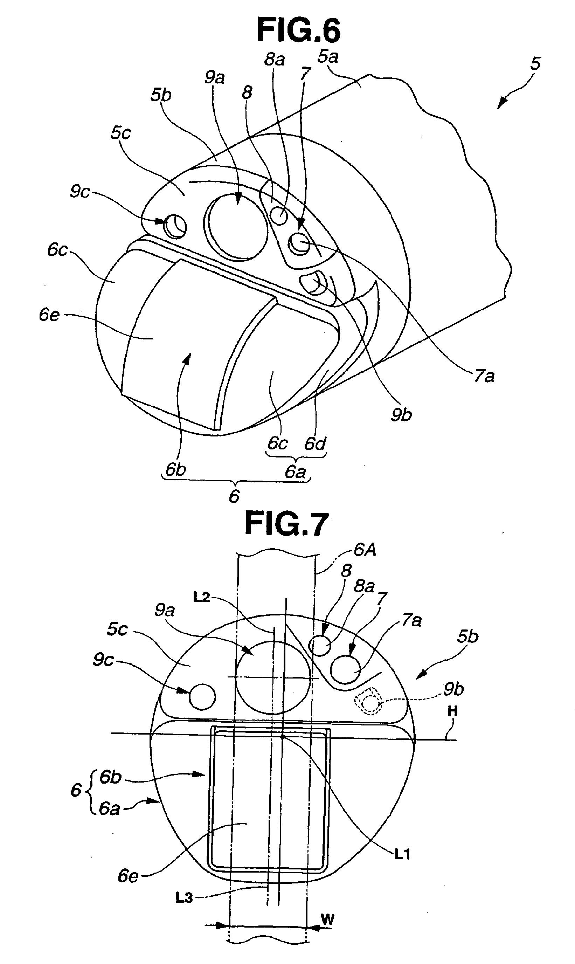

Furthermore, the ultrasound endoscope 5 is provided with an ultrasound transducer portion 6 for obtaining acoustic image data generated by ultrasound waves in a distal end rigid portion 5b of an endoscope insertion portion 5a as shown in FIG 6. The ultrasound transducer portion 6 is composed of a receptacle in the form of a nosepiece 6a and an ultrasound transreceiver portion 6b. The ultrasound transreceiver portion 6b is integrally positioned in a notch portion formed substantially in the center of the nosepiece 6a.

The ultrasound transducer portion 6 is provided with a convex ultrasound transreceiver portion, and forms an ultrasound observation region 6A that scans in a diagonal forward direction relative to the direction of the insertion axis (see FIG 12). In other words, the ultrasound transducer portion 6 has an ultrasound observation region 6A that scans in the diagonal forward direction.

A tissue contact surface 6c of a tissue contact portion 6d constituting the nosepiece 6a, and a transducer lens surface 6e of the ultrasound transreceiver portion 6b, are configured to protrude from a distal end surface 5c of the distal end rigid portion 5b.

An observation window 7a constituting an observation optics 7, an illumination window 8a constituting an illumination optics 8, a treatment instrument outlet (to be simply referred to as an outlet) 9a, an air/water feed nozzle 9b and an auxiliary water channel port 9c and the like are provided in the distal end surface 5c of the distal end rigid portion 5b. The outlet 9a is an opening for guiding a treatment instrument such as a puncture needle. The air/water feed nozzle 9b sprays a fluid such as water or air towards the observation window 7a. The auxiliary water channel port 9c feeds water in the anterior direction.

The ultrasound transducer portion 6 is configured by positioning a vertical center line L2 of the treatment instrument outlet 9a, and a vertical center line L3 of the transducer lens surface 6e of the ultrasound transreceiver portion 6b substantially on the same line.

The following provides an explanation of the procedure for transendoscopically introducing the positioning apparatus 1 into the jejunum and positioning the marker portions 21 and 21E in the jejunum as indicated with the broken lines in FIG 5.

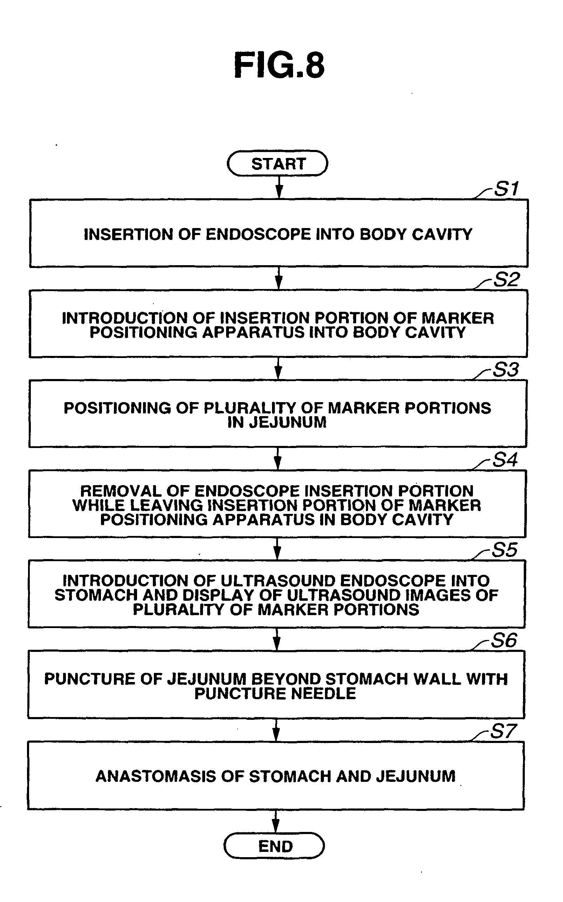

First, a surgeon introduces the endoscope 10 into a body cavity as indicated in step S 1 of FIG 8. More specifically, the endoscope insertion portion 11 of the endoscope 10 is introduced into a body cavity through the oral cavity or nasal cavity. The surgeon then passes the distal end rigid portion 14 of the endoscope insertion portion 11 through the esophagus and stomach while observing an endoscopic image not shown, and inserts until reaching a target site in the form of the duodenum as shown in FIG 5. The procedure subsequently proceeds to step S2.

The surgeon introduces the insertion portion of the marker positioning apparatus into a body cavity as indicated in step S2. Namely, the surgeon inserts the insertion portion 2a of the positioning apparatus 1 configured in the manner previously described into a treatment instrument channel (not shown) as indicated by the arrow through the treatment instrument inlet 12e as shown in FIG 9A. At this time, the surgeon confirms whether or not the distal end portion of the insertion 2a is displayed in the endoscopic image. The distal end portion of the insertion portion 2a is displayed in the endoscopic image as a result of the distal end surface of the insertion portion 2a being guided to the body cavity as indicated with the double-dashed line shown in the drawing. Subsequently, the surgeon inserts the insertion portion 2a deeper into the body cavity only by the distance to a predetermined site of the jejunum obtained from a preliminarily acquired diagnostic image generated by CT or MRI. At this time, the surgeon performs insertion while reading the scale 2c provided on the outer surface of the insertion portion 2a a shown in FIG. 9B.

The surgeon then discontinues further insertion of the insertion portion 2a once the distal end surface of the insertion portion 2a has been judged to have reached a preset target protruding position in the jejunum by reading the scale 2c. As a result, the distal end surface of the insertion portion 2a is positioned at the target protruding position as shown in FIG 10A. The procedure subsequently proceeds to step S3.

The surgeon then positions a plurality of the marker portions in the jejunum as indicated in step S3. Namely, the surgeon performs an extruding operation for positioning the second projection 3h by engaging with the circular indentation 2i instead of the first projection 3g positioned by engaging with the circular indentation 2i by performing a first stage of an extruding operation on the extruding member 3. At this time, the surgeon disengages the operation thread 23 positioned in the slit 3m from the slit 3m. The surgeon then instructs an assistant to pull the operation thread 23 towards the handheld side to prevent the operation thread 23 from being pulled into the gap between the marker guiding portion 3 a and the insertion portion 2a.

Accompanying the extrusion operation on the extruding member 3 performed by the surgeon, the first projection 3g is disengaged from the circular indentation 2i and the second projection 3h moves toward the circular indentation 2i as indicated by the arrow. In addition, the extruding surface 3d of the marker guiding portion 3a moves towards the direction of the distal end surface of the insertion portion 2a.

Whereupon, the marker portion 21E contacting the extruding surface 3d is moved towards the direction of the distal end surface of the insertion portion 2a in opposition to the resiliency of the marker portion 21E. Namely, accompanying movement of the extruding surface 3d, both the marker portion 21E and the marker portion 21 contacting the marker portion 21E housed on the distal end side thereof move towards the direction of the distal end surface of the insertion portion 2a in opposition to the resiliency thereof.

As a result, the marker portion 21 housed on the distal end side in the marker housing space portion 4 is gradually pushed into the jejunum. At this time, the marker portion 21 gradually returns to its ring shape due to the resiliency of the marker portion 21. When the second projection 3h has reached the vicinity of the circular indentation 2i, the marker portion 21 returns to the shape of a ring and catches on a circular fold of the jejunum where it is fixed in position. Subsequently, as shown in FIG 10B, the first stage of the extruding operation on the extruding member 3 is discontinued when the second projection 3h has been positioned by being engaged in the circular indentation 2i. As a result, the distal end side marker portion extrusion step is completed.

Subsequently, the surgeon fixes the marker 21E remaining in the marker housing space portion 4 at a predetermined location in the jejunum. In order to accomplish this, the surgeon performs a hand-side operation of retracting the distal end surface of the insertion portion 2a located at a target protruding position by a distance C. In other words, the surgeon pulls back the insertion portion 2a towards the handheld side while reading the scale 2c provided on the outer surface of the insertion portion 2a. Namely, an operation for retracting the insertion portion 2a is performed. Whereupon, accompanying retraction of the insertion portion 2a, the connecting threads 22 housed in the marker housing space portion 4 are gradually pulled out into the jejunum.

Once the surgeon has judged that the distal end surface of the insertion portion 2a has retracted by predetermined distance C from the preset target protruding position in the jejunum as shown in FIG 10C, the surgeon discontinues pulling the insertion portion 2a back towards the handheld side. As a result, the insertion portion retraction step is completed.

Subsequently, the surgeon performs a second stage of the extruding operation on the extruding member 3. Namely, the engaged state of the second projection 3h positioned by engaging with the circular indentation 2i is released, and the contact surface 3k moves towards the proximal end surface of the barrel flange portion 2e. In addition, the extruding surface 3d of the marker guiding portion 3a moves towards the direction of the distal end surface of the insertion portion 2a.

Whereupon, the marker portion 21E contacting the extruding surface 3d is moved towards the direction of the distal end surface of the insertion portion 2a in opposition to the resiliency of the marker portion 21E. As a result, the marker portion 21E remaining in the marker housing space portion 4 is gradually pushed out into the jejunum. At this time, the marker portion 21E gradually becomes circular due to the resiliency of the marker portion 21E. As shown in FIG 10C, the second stage of the extruding operation on the extruding member 3 is completed by contacting the contact surface 3k with the proximal end surface of the barrel flange portion 2e. As a result, the proximal end side marker portion extrusion step is completed.

Whereupon, as shown in FIG 11, the marker portion 21E catches on a circular fold of the jejunum by deforming into the shape of a ring and is fixed in position. As a result, positioning of the marker portions 21 and 21E in the jejunum is completed. The procedure subsequently proceeds to step S4.

The surgeon removes the endoscope insertion portion 11 of the endoscope 10 from the body so as to allow the insertion portion 2a of the positioning apparatus 1 to remain in the body cavity as indicated in step S4.

In this manner, the marker housing space portion is provided in the positioning apparatus and the two marker portions are housed in the marker housing space portion with the apparatus body and the extruding member constituting the positioning apparatus. The insertion portion of the apparatus body constituting the positioning apparatus is transendoscopically inserted into a desired site in a body cavity with the marker portions housed in the marker housing space portion. Subsequently, an operation is performed consisting of pushing out the marker guiding portion of the extruding member constituting the positioning apparatus in a stepwise manner. As a result, the two marker portions can be positioned at a desired site in a state of being mutually separated.

The following provides an explanation of a procedure for introducing the ultrasound endoscope 5 into the stomach and puncturing the jejunum through the stomach wall under the guidance of the ultrasound endoscope.

As indicated in step S5, the surgeon introduces the endoscope insertion portion 5a of the ultrasound endoscope 5 into the stomach from, for example, the oral cavity, and displays an endoscopic image on an endoscope observation screen of an ultrasound monitoring apparatus not shown. As shown in FIG. 12, the ultrasound transducer portion 6 is pressed against the stomach wall in the vicinity of a target site A of the stomach. Subsequently, the surgeon begins ultrasound observation by supplying an ultrasound wave transmitting medium in the form of, for example, sterile water, and displays an ultrasound tomographic image on an ultrasound observation screen of an ultrasound monitoring apparatus not shown. Here, the surgeon operates a bending operation knob and the like not shown of the ultrasound endoscope 5 to display the marker portions 21 and 21E in the ultrasound tomographic image.

As a result of this hand-side operation, two bright points 25A and 25B and bright points 25C and 25D separated by a distance equal to the diameter are displayed for each of the marker portions 21 and 21 E in an ultrasound tomographic image 50 as shown in FIG 13 by capturing the marker portions 21 and 21 A in the ultrasound monitoring region 6A as shown in FIG 12. At this time, the surgeon confirms whether or not blood vessels are present in the ultrasound tomographic image 50. The procedure subsequently proceeds to step S6.

In step S6, the surgeon punctures the jejunum beyond the stomach wall with a puncture needle. In order to accomplish this, the surgeon measures the distance to the jejunum while confirming the absence of blood vessels along the puncture route, sets the insertion distance based on that distance, and then advances the puncture needle toward the jejunum beyond the stomach wall. As a result, the tip of the puncture needle not shown reaches the range demarcated by the bright points 25A, 25B, 25C and 25D. Subsequently, the surgeon performs gastrojejunal anastomosis for anastomosing the stomach and jejunum by inserting a suturing device and the like by means of the puncture needle as indicated in step S7. Following completion of anastomosis, the suturing device, the ultrasound endoscope and the like are removed from the body cavity.

In this manner, at least a pair of the marker portions is positioned in advance in the jejunum, followed by depicting the marker portions positioned in the jejunum in the ultrasound tomographic image with the ultrasound endoscope. As a result, the surgeon is able to specify a target site of the jejunum beyond the stomach wall. Thus, the surgeon:is able to specify a target site of the jejunum in the ultrasound tomographic image and then safely and reliably perform puncture when transgastrically puncturing the jejunum with a puncture needle.

Furthermore, a marker withdrawal instrument 30 shown in FIG. 14 is used when withdrawing the marker portions 21 and 21 E positioned in the jejunum.

As shown in FIG 14, the marker withdrawal instrument 30 is in the form of a long, narrow flexible tube body. The marker withdrawal instrument 30 is provided with a through hole 31 through which the operation thread 23 is passed, and is provided with a withdrawal space 32 capable of housing a plurality, for example two, of the marker portions 21 and 21 E on the distal end side thereof.

The following provides an explanation of a procedure for withdrawing the marker portions.

When withdrawing the marker portions 21 and 2 1 E positioned in the jejunum, the user first connects the distal end of a guide thread 34 inserted in advance into the marker withdrawal instrument 30 to the proximal end of the operation thread 23. The user then pulls on the guide thread 34 to guide the end portion of the operation thread 23 in the through hole 31 through a distal end side opening of the marker withdrawal instrument 30 in the form of an opening 32a of the withdrawal space 32. The user then leads the end portion of the operation thread 23 through a proximal end side opening 33 of the marker withdrawal instrument 30 to the outside.

Next, the user introduces the marker withdrawal instrument 30 into a body cavity with the opening 32a of the marker withdrawal instrument 30 on the distal end side by using the operation thread 23 leading out of the body cavity as a guide. At this time, the user adjusts the inserted amount of the marker withdrawal instrument 30 by checking a scale (not shown) provided on the outer surface of the marker withdrawal instrument 30.

Next, the user pulls the operation thread 23 extending from proximal end side opening 33 of the marker withdrawal instrument 30 towards the user when the amount of insertion of the marker withdrawal instrument 30 has been judged to have reached the vicinity of the marker portion 21E. Whereupon, as shown in FIG 15, the operation thread 23 is moved in the direction of the arrow, and accompanying this movement of the operation thread 23 in the direction of the arrow, the marker portion 21 E moves so as to be pulled into the withdrawal space 32. As a result of the user further pulling the operation thread 23 towards the user, the marker portion 21 E gradually deforms after which the marker portion 2 1 E is housed in the withdrawal space 32 in opposition to the resiliency of the marker portion 21E.

Here, the user continues to pull the operation thread 23 towards the user. Whereupon, the marker portion 21E is further introduced into the back of the withdrawal space 32 in opposition to the resiliency of the marker portion 21E. Whereupon, as shown in FIG 16, the marker portion 21E contacts a back wall 32a of the withdrawal space 32 and pulling of the operation thread 23 towards the user is discontinued. As a result, the marker portion 21 E, the connecting threads 22 and the marker portion 21 are housed in the withdrawal space 32.

Subsequently, the user removes the marker withdrawal instrument 30 from the body cavity with the marker portion 21 E, the connecting threads 22 and the marker portion 21 housed in the withdrawal space 32. As a result, the procedure for withdrawing the marker portions 21 and 21E is completed.

Furthermore, in the present embodiment, the procedure for withdrawing the marker portions 21 and 21 E is described as being performed using the marker withdrawal instrument 30. However, the marker portion 21 E and the like may also be withdrawn in the marker housing space portion 4 by having the distal end of the insertion portion 2a of the positioning apparatus 1 preliminarily implanted in the stomach, for example, reinserting the insertion portion 2a to the vicinity of the marker portion 21 E by using the operation thread 23 as a guide as necessary, and pulling the operation thread 23 in the manner described above.

In addition, the withdrawal procedure can be made unnecessary by forming the marker portions 21 and 21 E, the connecting threads 22 and the operation thread 23 from a bioabsorbable material such as bioabsorbable resin. In this case, the procedure can be performed with a single ultrasound endoscope without having to change endoscopes by following steps S1 to S4 and steps S5 to S7 in FIG 8.

Moreover, the configuration of the organ anastomosis markers is not limited to that described above, but rather may be configured as shown in FIGS. 17 and 18.

As shown in FIG 17, a marker 40 of the present embodiment is composed of a plurality, for example two, of marker portions 41, the single string-like connecting thread 22, and the operation thread 23. The connecting thread 22 is a connecting member that connects the two marker portions 41. The marker portions 41 are made of an elastic member that reflects ultrasound waves in the form of a metal such as tungsten, a resin such as Grilamid, or an ultra-flexible metal such as an Ni-Ti-based alloy, and are circular in the no-load state as shown in the drawing.

The connecting threads 22 are connected to one end 41a of each of the marker portions 41 so that the interval between the two marker portions 41 is a preset interval. The operation thread 23 is fixed only to one of the marker portions 41. The specific fixed position of the operation thread 23 is the other end 41b of one of the marker portions 41. Furthermore, in the present embodiment, the marker portion 41 to which the operation 23 thread is fixed is indicated with reference symbol 41 E.

As shown in FIG 18, the two marker portions 41 and 41 E of the marker 40 are housed in a marker housing space portion 4A in a state of respectively being stretched linearly in opposition to the resiliency thereof. More specifically, the marker portions 41 and 41E are housed in the marker housing space portion 4A in the manner described below.

First, in the state in which the operation thread 23 has been extended towards the handheld side, the marker portion 41E to which the operation thread 23 is fixed among the two marker portions 41 and 41E is positioned in the marker housing space portion 4A as shown in FIG. 18. At this time, the marker portion 41E is linearly deformed in opposition to the resiliency of the marker portion 41 E. Subsequently, a worker draws the operation thread 23 close to the handheld side and contacts the marker portion 41E with the extruding surface 3d of the marker guiding portion 3a. The worker houses the connecting threads 22 in the marker housing space portion 4A while maintaining this contacted state together with positioning the other marker portion 41 in the marker housing space portion 4A by linearly deforming the marker portion 41 in opposition to the resiliency thereof. As a result, a positioning apparatus 1A has a configuration in which the two marker portions 41 and 41 E are housed in the marker housing space portion 4A.

In a marker provided with marker portions that linearly deform in opposition to the resiliency thereof as in the present embodiment, the diameter dimension of the space in which the marker portions are housed can be narrowed, and narrowing of the insertion portion constituting the apparatus body can be realized. Other actions and effects are similar to those of the previously described embodiment.

Having described the preferred embodiments of the invention referring to the accompanying drawings, it should be understood that the present invention is not limited to those precise embodiments, and various changes and modifications thereof could be made by one skilled in the art without departing from the scope of the invention as defined in the appended claims.

## Claims

1. An organ anastomosis marker (20) configured to be positioned by a marker positioning apparatus (1) having an apparatus body (2), comprising:
two marker portions (21) formed of an elastic member that is configured to reflect ultrasound waves, each of the marker portions (21) having, in a no-load state, a ring-shape;
**characterized in that** the marker (20) further comprises:
a pair of string-like connecting members (22) that connects the two marker portions (21) which are adjacent to each other at a preset interval, wherein one end of each of the connecting members (22) is connected to preset positions of one of the two marker portions (21), and the other ends of the connecting members (22) are connected to preset positions of the other of the two marker portions (21); and
a string-like member (23) having a distal end fixed to a proximal one (21E) of the two marker portions (21) and a proximal end having a length configured to lead outside the apparatus body (2).

2. The organ anastomosis marker (20) according to claim 1, wherein the marker portions (21) are made of metal, and a roughness area (21a) that reflects ultrasound waves is provided on the surface of the marker portions (21).

3. The organ anastomosis marker (20) according to claim 1, wherein the marker portions (21) are made of resin, and an ultrasound wave-reflecting substance is mixed in a resin member for forming the marker portions (21).

4. A marker positioning apparatus (1) comprising:
the organ anastomosis marker (20) as defined in claim 1;
an apparatus body (2) having: a flexible tube body (2a) which is provided with a housing portion (4) for housing the marker portions (21) constituting the organ anastomosis marker (20) in the distal end thereof and which can be inserted into a treatment instrument channel (17) of an endoscope (10); and a barrel (2b) connected to the proximal end portion of the tube body (2a); and
an extruding member (3) having: a flexible marker guiding portion (3a) slidably positioned in a through hole (2f) of the tube body (2a) constituting the apparatus body (2) and provided with a through hole (3c) into which the string-like member (23) constituting the organ anastomosis marker (20) is inserted, the distal end surface thereof being configured in the form of an extruding surface (3d) for pushing out the marker portions (21) housed in the housing portion (4) on the distal end of the tube body (2a) from the housing portion; and a tube (3b) slidably positioned in the through hole (2g) of the barrel (2b) to which the proximal end portion of the marker guiding portion (3a) is connected.

5. The marker positioning apparatus (1) according to claim 4, wherein the tube body (2a) constituting the apparatus body (2) is provided with a scale (2c) on the outer surface thereof.

6. The marker positioning apparatus (1) according to claim 4, wherein the barrel (2b) constituting the apparatus body (2) is provided with a circular indentation (2i) in the inner surface of the through hole (2g) of the barrel (2b), and the tube (3b) constituting the extruding member (3) has a plurality of projections (3g, 3h) on the outer surface of the tube (3b) that are positioned by engaging with the indentation (2i), while also having a contact portion that contacts the proximal end side (2e) of the barrel (2b).

7. The marker positioning apparatus (1) according to claim 6, wherein a projection (3g) among the plurality of the projections (3g, 3h) which is located farest away from the contact surface (3k) of the contact portion determines a positioning location of the extruding surface (3d) in the tube body, and other projections (3h) and the contact surface (3k) determine a distance over which the extruding surface (3d) moves in a stepwise manner.

## Patentansprüche

1. Organ-Anastomose-Marker (20), der dazu eingerichtet ist, durch eine Marker-Positionierungsvorrichtung (1), die einen Vorrichtungskörper (2) aufweist, positioniert zu werden, aufweisend:
zwei Marker-Teile (21), die aus einem elastischen Element gebildet sind, das dazu eingerichtet ist, Ultraschallwellen zu reflektieren, wobei jedes der Marker-Teile (21) in einem unbelasteten Zustand eine Ringform aufweist:
**dadurch gekennzeichnet, dass** der Marker (20) ferner aufweist:
ein Paar schnurartiger Verbindungselemente (22), das die zwei Marker-Teile (21) verbindet, die benachbart zu einander mit einem vorbestimmten Intervall sind, wobei ein Ende von jedem der Verbindungselemente (22) mit vorbestimmten Positionen von einem der zwei Marker-Teile (21) verbunden ist, und die anderen Enden der Verbindungselemente (22) mit vorbestimmten Positionen des anderen der zwei Marker-Teile (21) verbunden sind; und
ein schnurartiges Element (23) mit einem Distalende, das an einem proximalen (21E) der zwei Marker-Teile (21) befestigt ist, und einem Proximalende mit einer Länge, die dazu eingerichtet ist, außerhalb des Vorrichtungskörpers (2) geführt zu werden.

2. Organ-Anastomose-Marker (20) nach Anspruch 1, bei dem die Marker-Teile (21) aus Metall bestehen, und ein rauer Bereich (21a), der Ultraschallwellen reflektiert, auf der Oberfläche der Marker-Teile (21) bereitgestellt ist.

3. Organ-Anastomose-Marker (20) nach Anspruch 1, bei dem die Marker-Teile (21) aus Harz bestehen, und eine Ultraschallwellen-reflektierende Substanz in einem Harzelement gemischt ist, um die Marker-Teile (21) zu bilden.

4. Marker-Positionierungsvorrichtung (1) aufweisend:
den Organ-Anastomose-Marker (20) wie in Anspruch 1 definiert;
einen Vorrichtungskörper (2) aufweisend: einen flexiblen Röhrenkörper (2a), der mit einem Gehäuseteil (4) zum Aufnehmen der Marker-Teile (21), die den Organ-Anastomose-Marker (20) bilden, in dem Distalende davon bereitgestellt ist, und der in einen Behandlungsinstrumentenkanal (17) eines Endoskops (10) eingeführt werden kann; und einer Trommel (2b), die mit dem proximalen Endteil des Röhrenkörpers (2a) verbunden ist; und
ein extrudierendes Element (3) aufweisend: ein flexibles Marker-Führungsteil (3a), das verschiebbar in einem Durchgangsloch (2f) des Röhrenkörpers (2a) positioniert ist, und das den Vorrichtungskörper (2) bildet und bereitgestellt ist mit einem Durchgangsloch (3c), in das das schnurartige Element (23), das den Organ-Anastomose-Marker (20) bildet, eingeführt ist, wobei die Distalendfläche davon in der Form einer Extrudierungsfläche (3d) zum Herausdrücken des Marker-Teils (21) konfiguriert ist, das in dem Gehäuseteil (4) an dem Distalende des Röhrenkörpers (2a) von dem Gehäuseteil aufgenommen ist; und eine Röhre (3b), die verschiebbar in dem Durchgangsloch (2g) der Trommel (2b), an der das Proximalendteil des Marker-Führungsteils (3a) verbunden ist, positioniert ist.

5. Marker-Positionierungsvorrichtung (1) nach Anspruch 4, bei der der Röhrenkörper (2a), der den Vorrichtungskörper (2) bildet, mit einer Skala (2c) an dessen äußerer Oberfläche bereitgestellt ist.

6. Marker-Positionierungsvorrichtung (1) nach Anspruch 4, bei der die Trommel (2b), die den Vorrichtungskörper (2) bildet, mit einer runden Vertiefung (2i) in der inneren Fläche des Durchgangsloch (2g) der Trommel (2b) bereitgestellt ist, und die Röhre (3b), die das extrudierende Element (3) bildet, eine Mehrzahl von Vorsprüngen (3d, 3h) auf der äußeren Oberfläche der Röhre (3b) aufweist, die positioniert sind durch in Eingriff bringen mit der Vertiefung (2i), wobei auch ein Kontaktteil existiert, das die Proximalendseite (2e) der Trommel (2b) kontaktiert.

7. Marker-Positionierungsvorrichtung (1) nach Anspruch 6, bei der ein Vorsprung (3g) unter der Mehrzahl von Vorsprüngen (3g, 3h), der am entferntesten von der Kontaktfläche (3k) des Kontaktteils angeordnet ist, einen Positionierungsort der Extrudierungsfläche (3d) in dem Röhrenkörper bestimmt, und andere Vorsprünge (3h) und die Kontaktfläche (3k) eine Distanz bestimmen, über sich die Extrudierungsfläche (3d) schrittweise bewegt.

## Revendications

1. Marqueur (20) d'anastomose d'organes configuré pour être positionné par un appareil (1) de positionnement de marqueur ayant un corps (2) d'appareil, comprenant :
deux parties (21) de marqueur constituées d'un élément élastique qui est configuré pour réfléchir les ondes ultrasonores, chacune des parties (21) de marqueur ayant, dans un état sans charge, une forme d'anneau ;
**caractérisé en ce que** le marqueur (20) comprend en outre :
une paire d'éléments (22) de connexion de type fil qui connectent les deux parties (21) de marqueur qui sont adjacentes l'une à l'autre à un intervalle prédéfini, dans lequel une extrémité de chacun des éléments (22) de connexion est connectée sur des positions prédéfinies d'une des deux parties (21) de marqueur, et les autres extrémités des éléments (22) de connexion sont connectées sur des positions prédéfinies de l'autre des deux parties (21) de marqueur ; et
un élément (23) de type fil ayant une extrémité distale fixée à une extrémité proximale (21E) des deux parties (21) de marqueur et une extrémité proximale ayant une longueur configurée pour conduire hors du corps (2) d'appareil.

2. Marqueur (20) d'anastomose d'organes selon la revendication 1, dans lequel les parties (21) de marqueur sont constituées de métal, et une zone de rugosité (21a) qui réfléchit les ondes ultrasonores est prévue sur la surface des parties (21) de marqueur.

3. Marqueur (20) d'anastomose d'organes selon la revendication 1, dans lequel les parties (21) de marqueur sont constituées de résine, et une substance réfléchissant les ondes ultrasonores est mélangée dans un élément de résine pour former les parties (21) de marqueur.

4. Appareil (1) de positionnement de marqueur comprenant :
le marqueur (20) d'anastomose d'organes selon la revendication 1 ;
un corps (2) d'appareil ayant : un corps (2a) de tube flexible qui est prévu avec une partie (4) de logement pour loger les parties (21) de marqueur constituant le marqueur (20) d'anastomose d'organes dans l'extrémité distale de celui-ci et qui peut être inséré dans un canal (17) d'instrument de traitement d'un endoscope (10) ; et un barillet (2b) connecté à la partie d'extrémité proximale du corps (2a) de tube ; et
un élément (3) d'extrusion ayant : une partie (3a) flexible de guidage de marqueur positionnée de façon coulissante dans un trou traversant (2f) du corps (2a) de tube constituant le corps (2) d'appareil et prévue avec un trou traversant (3c) dans lequel l'élément (23) de type fil constituant le marqueur (20) d'anastomose d'organes est inséré, la surface d'extrémité distale de celui-ci étant configurée sous la forme d'une surface (3d) d'extrusion pour pousser les parties (21) de marqueur logées dans la partie (4) de logement sur l'extrémité distale du corps (2a) de tube hors de la partie de logement ; et un tube (3b) positionné de façon coulissante dans le trou traversant (2g) du barillet (2b) auquel la partie d'extrémité proximale de la partie (3a) de guidage de marqueur est connectée.

5. Appareil (1) de positionnement de marqueur selon la revendication 4, dans lequel le corps (2a) de tube constituant le corps (2) d'appareil est prévu avec une échelle (2c) sur la surface extérieure de celui-ci.

6. Appareil (1) de positionnement de marqueur selon la revendication 4, dans lequel le barillet (2b) constituant le corps (2) d'appareil est prévu avec une indentation (2i) circulaire dans la surface intérieure du trou traversant (2g) du barillet (2b), et le tube (3b) constituant l'élément (3) d'extrusion a une pluralité de projections (3g, 3h) sur la surface extérieure du tube (3b) qui sont positionnées par engagement avec l'indentation (2i), tout en ayant aussi une partie de contact qui est en contact avec le côté d'extrémité proximale (2e) du barillet (2b).

7. Appareil (1) de positionnement de marqueur selon la revendication 6, dans lequel une projection (3g) parmi la pluralité de projections (3g, 3h) qui est située la plus éloignée de la surface (3k) de contact de la partie de contact détermine un emplacement de positionnement de la surface (3d) d'extrusion dans le corps de tube, et d'autres projections (3h) et la surface (3k) de contact déterminent une distance sur laquelle la surface (3d) d'extrusion se déplace de manière pas à pas.
